# EUROPEAN PATENT APPLICATION

(11) **EP 4 765 137 A1**
(43) Date of publication of application: **24.06.2026**
(21) Application number: 24221794.1
(22) Date of filing: 19.12.2024
(51) Int. Cl.: G16H 10/60, G16H 40/67, G16H 50/20

(54) **PATIENT ENVIRONMENT INFORMATION PROVIDING SYSTEM**

(71) Applicant: Koninklijke Philips N.V., 5656 AE Eindhoven (NL)
(72) Inventor: VOGTMEIER, Gereon, Eindhoven (NL); WEISS, Steffen, Eindhoven (NL); HELLE, Michael Günter, Eindhoven (NL); VON BERG, Jens, Eindhoven (NL)
(74) Representative: Philips Intellectual Property & Standards

(57) **Abstract**

A patient environment information providing system is provided that comprises a data collection device, a data synchronization device, and a data diagnose support device. The data collection device is configured to provide a data provider access to the system via at least one on-line user interface for inputting data that is relevant to the event from a plurality of information sources. The data synchronization device is configured to analyze the input data to obtain synchronizing information and to synchronize the input data based on the synchronizing information to provide a support data set for the patient and the event. The data diagnose support device is configured to provide a data diagnose support interface to at least one patient environment information obtaining device to provide the support data set for the patient and the event to the at least one patient environment information obtaining device.

## Description

### FIELD OF THE INVENTION

The invention relates to a patient environment information providing system, to a patient environment information obtaining device, to a patient environment information providing method, and to a patient environment information obtaining method.

### BACKGROUND OF THE INVENTION

Medical imaging refers to several different technologies that are used to view the human body in order to diagnose, monitor, or treat medical conditions. The medical image is one source of information, but it may be necessary to have additional information. However, in case of e.g., an accident the patient might not be able to give this information. The police and/or ambulance people might have only limited additional information. Even if this information would be available, it is often very hard to have it available for the doctor at the time of doing the diagnose.

### SUMMARY OF THE INVENTION

There may be a need in the art to provide additional information about the circumstances of an event when a patient is diagnosed.

The object of the present invention is solved by the subject matter of the independent claims where further embodiments are incorporated in the dependent claims. It should be noted that the following described aspect of the invention equally applies to the patient environment information providing system, to the patient environment information obtaining device, to the patient environment information providing method, and to the patient environment information obtaining method.

According to a first aspect of the present invention, there is provided a patient environment information providing system for managing and collecting information about a circumstance of an event having an impact on a health status of a patient. The system comprises a data collection device, a data synchronization device, and a data diagnose support device. The data collection device is configured to provide a data provider access to the system via at least one on-line user interface for inputting data that is relevant to the event from a plurality of information sources. The data synchronization device is configured to analyze the input data to obtain synchronizing information and to synchronize the input data from the plurality of information sources to the event based on the synchronizing information to provide a support data set for the patient and the event. The data diagnose support device is configured to provide a data diagnose support interface to at least one patient environment information obtaining device to receive a data diagnose support request therefrom, and in response to receiving the data diagnose support request, to provide the support data set for the patient and the event to the at least one patient environment information obtaining device.

Accordingly, the proposed patient environment information providing system may be provided with a data collection system, and a data synchronization tool with an interface to at least one patient environment information obtaining device, such as an imaging system and a diagnostic software that is used by the clinical staff inside the hospital, an ambulance, and/or a local medical center. The at least one patient environment information obtaining device may include e.g., imaging systems, image diagnostic suites, and medical devices. The proposed patient environment information providing system may allow a doctor for more precise diagnoses because of having additional relevant information.

In embodiments, the patient environment information providing system further comprises a database configured to store a plurality of case data sets comprising information about a plurality of events having an impact on a health status of one or more patients, each data set comprising event information and patient information.

In embodiments, in response to receiving the data support request, the data diagnose support device is configured to search in the database for one or more case data sets that could match the received data support request, and to perform one or more plausibility checks on the one or more case data sets.

In embodiments, the data synchronization device is further configured to analyze the input data to determine whether the input data contains personal information, and in response to determining that the input data contains personal information, the data synchronization device is configured to:
- not synchronize the input data containing personal information to the event;
- label the input data containing personal information which is only assessable when a privacy approval is available; or
- hide personal information contained in the input data, wherein the hidden personal information is only accessible when a privacy approval is available or for a certain authority.

For example, when pictures and/or videos are uploaded to the patient environment information providing system, an image processing algorithm or a face recognition algorithm may be used to detect personal information (e.g. license plates, human faces, other personal information, etc.) that should not be synced or require privacy approval. Such pictures and/or videos may be labeled and only be accessed when approval is available. Another possibility is to blur only the parts in the pictures and/or videos that show personal information and de-blur after approval is given or for certain authorities such as police.

In embodiments, the data synchronization device is further configured to assign a level of reliability to the input data in accordance with information resources associated with the input data.

For example, information from the first responder (e.g., police, firefighter, etc.) may be assigned with a higher level of reliability. Information from the people who knows the patient, such as friends and parents, may also be assigned with a higher level of reliability. The level of reliability may be indicated with a reliability score. In this way, it is possible to enlarge reliability of some input data with a higher reliability score.

In embodiments, the at least one on-line user interface comprises one or more of:
- a browser-based web user interface;
- an app installable on a mobile device; and
- a web portal.

In embodiments, information about the at least one on-line user interface is obtainable from one or more of the following:
- an image-based input providing information about the at least one on-line user interface in a visual, machine-readable form;
- a network-based input with auto popup screen on a mobile device e.g., when the mobile device is in an area of a care provider that supports the patient; and/or
- a beacon-based input with auto popup screen on a mobile device e.g., when the mobile device is in an area of ambulance.

In embodiments, the input data comprises one or more of:
- text information of the event with location information, time information, and/or a short description;
- first supportive data provided by a first aid provider;
- a picture;
- a video;
- a voice recording;
- weather information that is relevant to the event; and
- publicly available information about an event environment.

In embodiments, the synchronization information comprises one or more of:
- location information and/or time information of the input data;
- image information on a picture and/or on a video;
- information on a license plate of a vehicle;
- a dedicated Quick Response, QR, code on an ambulance, on a medical staff, on a police staff, and/or on a medical equipment;
- a wireless local network identifier;
- a cell ID of a mobile phone network; and
- an electronic finder.

In embodiments, the patient environment information providing system is a cloud-based system.

According to a second aspect of the present invention, there is provided a patient environment information obtaining device. The device comprises a user interface, a data diagnose support interface, and a processor. The user interface is configured to receive a user input indicative of a need for information about a circumstance of an event having an impact on a health status of a patient. The processor is configured to send, via the data diagnose support interface, a data diagnose support request to a system for managing and collecting information about a circumstance of an event having an impact on a health status of a patient, and to receive, via the data diagnose support interface, a support data set for the patient and the event from the system.

For example, the patient environment information obtaining device may comprise a user interface with an interactive element (e.g., a button). The interactive element may be activated by a user (e.g., a doctor) with a mouse, keyboard, finger, voice command, or other assistive technology. Once activated, the interactive element then performs an action, such as sending, via the data diagnose support interface, a data diagnose support request to a patient environment information providing system, and receiving, via the data diagnose support interface, a support data set for the patient and the event from the system.

In embodiments, the patient environment information obtaining device comprises one or more of:
- a medical imaging system;
- a hospital information system; and
- a mobile device.

According to a third aspect of the present invention, there is provided a patient environment information providing method for managing and collecting information about a circumstance of an event having an impact on a health status of a patient, the method comprising:
- providing a data provider access to the system via at least one on-line user interface for inputting data that is relevant to the event from a plurality of information sources;
- analyzing the input data to obtain synchronizing information and to synchronize the input data from the plurality of information sources to the event based on the synchronizing information to provide a support data set for the patient and the event; and
- providing a data diagnose support interface to at least one patient environment information obtaining device to receive a data support request, and to provide the support data set for the patient and the event to the at least one patient environment information obtaining device, in response to receiving the data support request.

According to a third aspect of the present invention, there is provided a patient environment information obtaining method, the method comprising:
- receiving, via a user interface, a user input indicative of a need for information about a circumstance of an event having an impact on a health status of a patient;
- sending, via the data diagnose support interface, a data diagnose support request to a system for managing and collecting information about a circumstance of an event having an impact on a health status of a patient; and
- receiving, via the data diagnose support interface, a support data set for the patient and the event from the system.

According to an aspect of the present invention, there is provided a computer program element, which, when being executed by at least one processing unit, is adapted to cause the processing unit to perform the method according to the first aspect or to perform the method according to the second aspect.

According to a fourth aspect, there is provided a computer readable medium having stored thereon the program element.

These and other aspects of the present invention will become apparent from and be elucidated with reference to the embodiments described hereinafter.

### BRIEF DESCRIPTION OF THE DRAWINGS

These and other aspects of the invention will be apparent from and elucidated further with reference to the embodiments described by way of examples in the following description and with reference to the accompanying drawings, in which
Fig. 1 schematically depicts an exemplary computer network environment for implementing embodiments of the present disclosure.
Fig. 2 schematically depicts a further exemplary computer network environment for implementing embodiments of the present disclosure.
Fig. 3 illustrates a flowchart describing an exemplary patient environment information providing method.
Fig. 4 illustrates a flowchart describing an exemplary patient environment information obtaining method.

It should be noted that the figures are purely diagrammatic and not drawn to scale. In the figures, elements which correspond to elements already described may have the same reference numerals. Examples, embodiments or optional features, whether indicated as non-limiting or not, are not to be understood as limiting the invention as claimed.

### DETAILED DESCRIPTION OF EMBODIMENTS

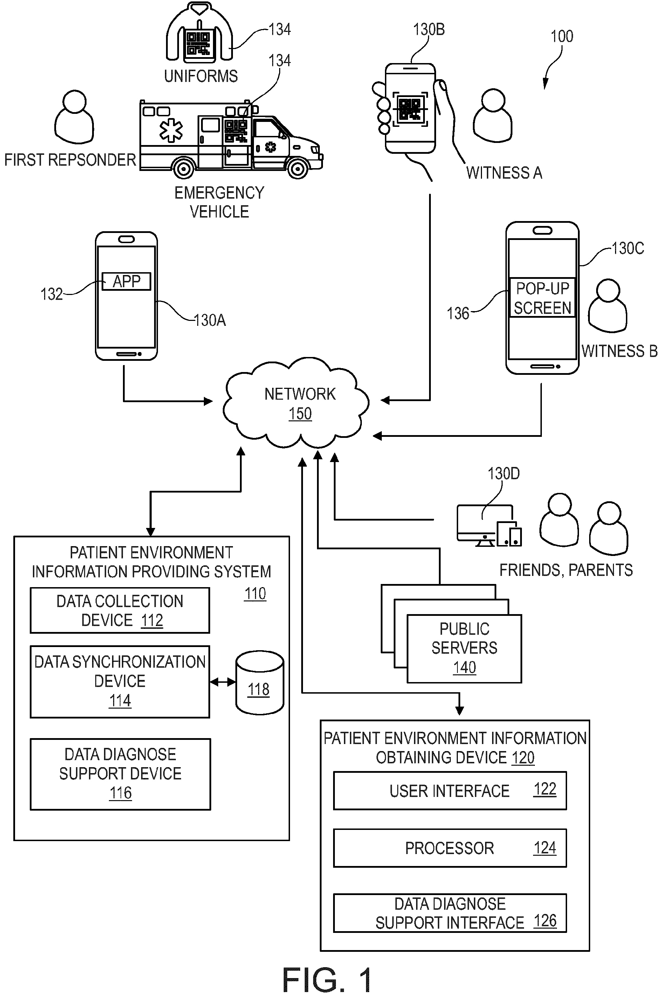

Fig. 1 schematically depicts an exemplary computer network environment 100 for implementing embodiments of the present disclosure. As illustrated, the computer network environment 100 includes a patient environment information providing system 110, a patient environment information obtaining device 120, a plurality of electronic communication devices 130, such as devices 130A-130D, one or more public servers 140, and a network 150.

The exemplary patient environment information providing system 110 of the illustrated example is configured to provide a data provider access to the system via at least one on-line user interface for inputting data that is relevant to the event from a plurality of information sources.

The components 112, 114, 116, and 118 of the exemplary patient environment information providing system 110 may be implemented as software modules or routines in a single software suit and run on a general purpose computing unit such as a server. Alternatively, the components 112, 114, 116, and 118 of the exemplary patient environment information providing system 110 may be arranged in a distributed architecture and connected in a suitable communication network.

Some or all components of the exemplary patient environment information providing system 110 may be arranged in hardware such as a suitably programmed FPGA (field-programmable-gate-array) or as hardwired IC chip. One or more features of the exemplary patient environment information providing system 110 disclosed herein may be configured or implemented as/with circuitry encoded within a computer-readable medium, and/or combinations thereof. Circuitry may include discrete and/or integrated circuitry, application specific integrated circuitry (ASIC), a system-on-a-chip (SOC), and combinations thereof., a machine, a computer system, a processor and memory, a computer program.

In a further example, some or all components of the exemplary patient environment information providing system 110 may be embodied as a collection of webservices which run on a cloud infrastructure. These webservices are accessible from various client devices through a thin client interface such as a web browser, a mobile app, or a desktop app. The user does not manage or control the underlying cloud infrastructure including network, servers, operating systems, storage, or even individual application capabilities, with the possible exception of limited user-specific application configuration settings. The web server may process incoming network requests over HTTP and several other related protocols for communication between the web browser and the web server unit. The layout of the webpage may use cascading Style Sheets (CSS) or Bootstrap. In an example, the GUI may have an Angular code base whose product is executable within a web browser. Accordingly, it may be visualized on every device providing a browser. The Angular framework may be used to create JavaScript and/or TypeScript code for the webpage. The patient environment information providing system 110 will be described in more detail below.

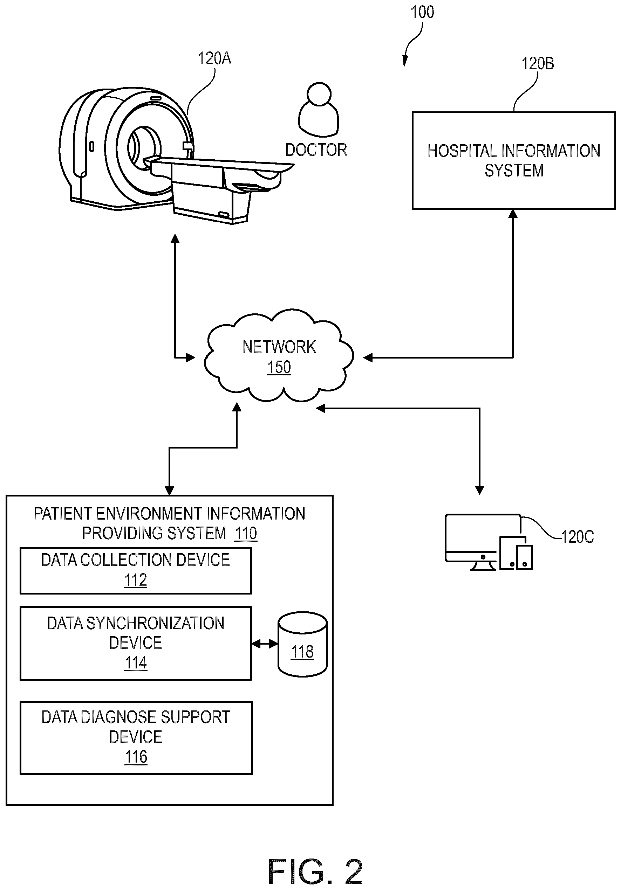

The patient environment information obtaining device 120 of the illustrated example is configured to receive a user input indicative of a need for information about a circumstance of an event having an impact on a health status of a patient. In response to receiving the user input, the patient environment information obtaining device 120 may send a data diagnose support request to the patient environment information providing system 110. This request may include e.g., patient data and case data. The patient data and case data may be obtained from patient record, and/or an entry field. The patient environment information obtaining device 120 may then receive additional information relating to the patient and the event from the patient environment information providing system 110. The patient environment information obtaining device 120 may be a software or a hardware dedicated to running said software. The software may comprise an interactive element (e.g., a button) in the user interface to request additional patient environment information. Exemplary patient environment information obtaining devices 120 are shown in Fig. 2 and will be described in more detail below.

The plurality of electronic communication devices 130, such as devices 130A-13D, may comprise an application configured to interface with the web service provided by the patient environment information providing system 110. The application may also be referred to as an "app" or an "software application", which refers to a computer program or group of programmes designed for end users. The terms shall encompass standalone applications, thin client applications, thick client applications, web-based applications, such as a browser, and other similar applications. For example, a web browser application at the plurality of electronic communication devices 130 may support interfacing with a web server application at the patient environment information providing system. Such a browser may use controls, plug-ins, or applets to support interfacing to the patient environment information providing system 110. The plurality of electronic communication devices 130 may use other customized programs, applications, or modules to interface with the patient environment information providing system 110. The plurality of electronic communication devices 130 may be desktop computers, laptops, handhelds, mobile devices, mobile telephones, servers, terminals, thin-clients, or any other computerized devices.

The patient environment information providing system 110 may retrieve publicly-available information from the one or more public servers 140. Exemplary publicly-available information may include, but is not limited to, weather information, traffic information, other events that happened near the site of the event, and/or other relevant information to explain what happened and how it could lead to the special situation for the patient. The one or more public servers 140 may comprise servers that provide weather information, traffic information, etc. The public servers 140 may include search engines, such as Google, Yahoo, etc. The patient environment information providing system 110 may use one or more search engines commonly available to perform an Internet search to identify websites and related servers that contain e.g., weather information, traffic information, other events that happened near the site of the event, etc. The patent environment information providing system 110 may receive the links and retrieve data from one or more of the links in the form of text, digital photographs, and digital video using techniques well known in the art to automatically scan web pages and retrieve such data.

The network 150 may be any communications network capable of supporting communications between the patient environment information providing system 110, the patient environment information obtaining device 120, the plurality of electronic communication devices 130, and the one or more public servers 140. The network 150 may be wired, wireless, optical, radio, packet switched, circuit switched, or any combination thereof. The network 150 may use any topology, and links of the network 150 may support any networking technology, protocol, or bandwidth such as Ethernet, DSL, cable modem, ATM, SONET, MPLS, PSTN, POTS modem, PONS, HFC, satellite, ISDN, Wi-Fi, WiMax, mobile cellular, any combination thereof, or any other data interconnection or networking mechanism. The network 150 may be an intranet, the Internet (or the World Wide Web), a LAN, WAN, MAN, or any other network for interconnecting computers. To support high volume and load, a distributed computing environment may be implemented by using networking technologies that may include, but are not limited to, TCP/IP, RPC, RMI, HHTP, Web Services (XML-RPC, JAX-RPC, SOAP, etc.).

In the exemplary computer network environment 100 shown in Fig. 1, an event may occur that has an impact on a health status of a patient. In an example, the event may be an accident, such as a ski accident or car accident. If the event happens, a first responder, such as emergency medical service workers, police officers, or firefighters may be deployed at the site of the event. As shown in Fig. 1, the first responder may have a mobile device, such as a mobile phone, with an app 132 residing thereon. The first responder may provide information about the circumstances of the event via the app 132, which may transmit the data to the patient environment information providing system 110 via secure, encrypted communications via the network 150. The special app 132 residing on the mobile device of the first responder may be used to initiate an emergency call, but also directly collect first supportive data, such as type of questions that have been asked, type of information that have been searched, pictures etc.

Besides the first responder, people who witnessed this event may also provide additional information. For example, as shown in Fig. 1, the first responders may have their vehicles, uniforms, and/or equipment (not shown) covered with a digital pattern, such as QR code 134 with additional information like "Want to help the patient - send relevant info to medical support QR". When a witness A scans the QR code 134 with his/her smartphone 130B, a message will pop up on the display. The message may provide an on-line user interface for inputting data that is relevant to the event. For example, after the witness A scanned the QR code 134, the witness A is directed to a browser-based web user interface or a web portal for inputting data that is relevant to the event. In some embodiments, the first responders may have their vehicles, and/or equipment (not shown) equipped with a Beacon, which is a small, Bluetooth device that broadcasts tiny radio signals around itself at low power consumption. Using Bluetooth technology, the beacon is able to estimate the position of the smartphones and interact with them by exchanging data and information. The witness B may receive notifications while he/she is in the area of the first responder's vehicle and/or equipment. The notifications 136 may pop up on the display of the mobile device 130C of the witness B. For example, the notifications may be "Want to help the patient - please click the link below to send relevant info to medical support". In some further examples, the first responder's vehicle may be equipped with a Wi-Fi network. When connecting to the Wi-Fi network, notifications may pop up on the display of the mobile device 130C of the witness B. For example, the notifications may be "Want to help the patient - please click the link below to send relevant info to medical support". In some implementations, it is possible to have the popup screen configurable. For example, it may be not always when in the area the message comes up. If the witness B has the app and wants to make an upload, the sync is made via "auto sync" and/or "ready to enter for this event".

Additional data from people knowing the patient, such as friends and parents, who have been informed about the event and want to give important information might be possible. For example, as shown in Fig. 1, the friends and the parents of the patient may be informed to provide additional information via a link to an on-line user interface, such as a web portal. The friends and parents may use their electronic devices 130D, such as mobile phones or personal computers, to input data relevant to the event via the on-line user interface. For example, the friends and parents may provide additional information, such as patient information (e.g., patient's age, patient's size, etc.), medical emergency information about the patient, contact who might have more information, address of doctor who normally takes care of the patient, etc.

The on-line user interface for inputting data related to the event may comprise one or more of a picture uploader user interface (UI) component, a video uploader UI component, and a text field. The picture uploader UI component is an interface designed to facilitate the uploading of pictures to the patient environment information providing system 110. For example, the first responder, the witness A, and/or witness B may upload pictures taken at the site of the event showing the situation of the patient and the environment. The video uploader UI component is an interface designed to facilitate the uploading of videos to the patient environment information providing system 110. For example, the first responder, the witness A, and/or the witness B may upload videos taken at the site of the event that might give additional information about the event. The witness A and/or the witness B may take the videos without expecting an accident to happen. Therefore, the videos may show how e.g., the injury of the patient occurred. The text field may allow users to enter text into the user interface, which is then transmitted to the patient environment information providing system 110. For example, it is possible to provide, via the text field, text information of the event (e.g., accident, ski accident, car accident, etc.) with location information (e.g., city, street, highway, mountain, ski track, etc.) and time information (e.g., day, time, etc.) and a short description of the event.

In addition to the user input, the patient environment information providing system 110 may retrieve publicly-available information from the one or more public servers 140. Exemplary publicly-available information may include, but is not limited to, weather information, traffic information, other events that happened near the site of the event, and/or other relevant information to explain what happened and how it could lead to the special situation for the patient.

All data that is entered via the different input interfaces will be collected by the patient environment information providing system 110, which sorts and links the data together to build a support data set for the patient and the dedicated event. In the example shown in Fig. 1, the exemplary patient environment information providing system 110 comprises a data collection device 112, a data synchronization device 114, and a data diagnose support device 116. Optionally, the patient environment information providing system 110 may further comprise a database 118. The exemplary patient environment information providing system may be a software, or hardware dedicated to running said software.

The data collection device 112 is configured to provide a data provider access to the system 110 via at least one on-line user interface for inputting data that is relevant to the event from a plurality of information sources. The at least one on-line user interface may comprise one or more of a browser-based web user interface, an app installable on a mobile device, and a web portal. For example, in Fig. 1 the data collection device 112 is configured to provide a data provider access to the system 110 via an app 132 for inputting data from the first responder. The data collection device 112 is configured to provide a data provider access to the system 110 via a web portal or a browser-based web user interface for inputting data from e.g., the witness A, the witness B, friends, and/or parents. The input data may comprise text information of the event with location information, time information, and/or a short description. The input data may comprise a picture taken at a site of the event. The input data may comprise a video taken at a site of the event. The input data may comprise a voice recording. The input data may comprise weather information that is relevant to the event, and publicly available information about an event environment. The input data may comprise any combination of the above-described exemplary input data.

The data synchronization device 114 is configured to analyze the input data to obtain synchronizing information and to synchronize the input data from the plurality of information sources to the event based on the synchronizing information to provide a support data set for the patient and the event. In other words, the data synchronization device 114 combines information from various information sources. Different synchronization mechanisms might be applicable. In an example, the synchronization information may comprise location information and/or time information of the input data. The location information may be obtained from e.g., Global Positioning System (GPS) data of pictures, network ID of the device when still close by and or simple text info about city, street, close to house number, or the other location information in the description. The location information of the input data may show whether the input data is collected at a location close to the event. The time information may be obtained from e.g., a time stamp of accident by first call for ambulance, arriving police, ambulance report and the time of event mentioned in the report of support data. Additionally, the time information may be obtained from data/time code in pictures, videos including daylight and darkness. In another example, the synchronization information may comprise image information on a picture and/or on a video. The image information may comprise metadata that comprises date, time, location, and other details hidden in the pictures and/or in the videos. The image information may comprise objects detected in the uploaded pictures and/or in the uploaded videos. The image information may show whether the pictures and/or videos are taken at the site of the event. In a further example, the synchronization information may comprise information on a license plate of a vehicle, such as ambulance, involved police car, patient's car, etc. The information on a license plate of a vehicle may be identified and obtained from the uploaded pictures and/or the uploaded videos. In a further example, the synchronization information may comprise a dedicated QR code on an ambulance, on a medical staff, on a police staff, and/or on a medical equipment. The dedicated QR code may be identified in the uploaded pictures and/or in the uploaded videos. In a further example, the synchronization information may comprise a cell ID of a mobile phone network. For example, mobile phone networks have cell IDs. The phone in the ambulance may measure the cells and store cell IDs of closest cells. Therefore, the person sending the information should be logged into one of the mentioned cells to show "sync" probability to be close to the event and in same area. This might be also possible via location and GPS information from an ambulance device and an informationgiver device. In another example, the synchronization information may comprise an electronic finder. The electronic finder may also be referred to as "find me token". Examples of the electronic finder may include e.g., Samsung Galaxy SmartTag and Apple Airtag. The small "find me tokens" for Apple/Android mobile phone devices may communicate via Bluetooth. The Bluetooth receiver of the phone in the ambulance and in phone of informationgiver might receive signals from the same token showing that both are in same region. In another example, the synchronization information may comprise a wireless local network identifier. Accordingly, the data synchronization device 114 may analyze one or more of the above-described exemplary synchronization information to determine whether to link the input data to the patient and the event to build a support data set for the patient and the event.

In some examples, the data synchronization device may be further configured to assign a level of reliability to the input data in accordance with information resources associated with the input data. For example, information from the first responder (e.g., police, firefighter, etc.) may be assigned with a higher level of reliability. Information from the people who knows the patient, such as friends and parents, may also be assigned with a higher level of reliability. The level of reliability may be indicated with a reliability score. In this way, it is possible to enlarge reliability of some input data with a higher reliability score.

In some examples, the data synchronization device may be further configured to analyze the input data to determine whether the input data contains personal information, and in response to determining that the input data contains personal information, the data synchronization device is configured to not synchronize the input data containing personal information to the event, to label the input data containing personal information which is only assessable when a privacy approval is available, or to hide personal information contained in the input data, wherein the hidden personal information is only accessible when a privacy approval is available or for a certain authority. For example, when pictures and/or videos are uploaded to the patient environment information providing system 110, an image processing algorithm or a face recognition algorithm may detect personal information (e.g. license plates, human faces, other personal information, etc.) that should not be synced or require privacy approval. Such pictures and/or videos may be labeled and only be accessed when approval is available. Another possibility is to blur only the parts in the pictures and/or videos that show personal information and de-blur after approval is given or for certain authorities such as police.

The database 118 is configured to store a plurality of case data sets comprising information about a plurality of events having an impact on a health status of one or more patients. Each data set comprises event information and patient information. The support data set for the patient and the event may be transmitted and stored in the database 118. In one example, the database 118 may be stored centrally in a data storage. In another example, the database may be stored in a secure and decentralized manner, such as in a cloud data storage.

The data diagnose support device 116 is configured to provide a data diagnose support interface to at least one patient environment information obtaining device 120 to receive a data diagnose support request therefrom, and in response to receiving the data diagnose support request, to provide the support data set for the patient and the event to the at least one patient environment information obtaining device 120.

For example, in response to receiving the data support request, the data diagnose support device 116 may be configured to search in the database for one or more case data sets that could match the received data support request, and to perform one or more plausibility checks on the one or more case data sets. For example, the data diagnose support device 116 may perform numerous checks, such as the checks on plausibility of time, location, patient's age, size of the patient, type of accident, etc. It may output the results of these checks as messages. It is also possible to specify the type (e.g., error, warning, or information) of the messages. For example, the data diagnose support device 116 may perform a plausibility check on the location information and/time information contained in the one or more case data sets and issue a message (e.g., warning) if the location information and/or the time information exceeds a predefined range. For example, for each diagnosis it is possible to specify the upper and lower age limit of the patient for whom the diagnosis can apply. The data diagnose support device 116 may perform a plausibility check on the patient's age contained in the one or more case data sets, and issue a message if the patient's age exceeds the upper age limit specified for the diagnosis or if the patient's age is below the lower age limit specified for the diagnosis. The message may be a warning message. In a further example, the data diagnose support device 116 may perform a plausibility check on the type of accident contained in the one or more case data sets and issue a message (e.g., warning). In one example, the data diagnose support device 116 may implement a matching algorithm to perform the check of plausibility. In another example, the data diagnose support device may implement a machine learning algorithm (such as neural networks) to perform the check of plausibility.

The patient environment information obtaining device 120 comprises a user interface 122, a processor 124, and a data diagnose support interface 124. The user interface 122 is configured to receive a user input indicative of a need for information about a circumstance of an event having an impact on a health status of a patient. The processor 124 is configured to send, via the data diagnose support interface 126, a data diagnose support request to a system for managing and collecting information about a circumstance of an event having an impact on a health status of a patient. The processor 124 is further configured to receive, via the data diagnose support interface 126, a support data set for the patient and the event from the system.
Fig. 2 illustrates an exemplary computer network environment including three exemplary patient environment information obtaining device 120.

In an example, the patient environment information obtaining device 120 may be embodied as, or in, an imaging system 120A. The imaging system 120A may be an X-ray system, a computed tomography (CT) system, a magnetic resonance imaging (MRI), or other imaging system. The components of the patient environment information obtaining device 120 may be implemented as software modules or routines in a single software suit and run on a general purpose computing unit such as a workstation associated with the imaging system 120A or a server computer associated with a group of imaging systems. Alternatively, the components of the patient environment information obtaining device 120 may be arranged in a distributed architecture and connected in a suitable communication network. The patient environment information obtaining device 120 may be fully integrated into the imaging system 120A. The patient environment information obtaining device 120 may comprise a user interface with an interactive element (e.g., a button). The interactive element may be activated by a user (e.g., a doctor) with a mouse, keyboard, finger, voice command, or other assistive technology. Once activated, the interactive element then performs an action, such as sending, via the data diagnose support interface, a data diagnose support request to a patient environment information providing system, and receiving, via the data diagnose support interface, a support data set for the patient and the event from the system. This request may include some patient data and case data, which may be obtained from patient record to start a search in the database 118. Case data sets that could fit may be checked on plausibility (e.g., time, location, patient's age, size of the patient, type of accident, etc.) to give feedback in case of an exact match or with a certain confidentiality level.

In another example, the patient environment information obtaining device 120 may be embodied as, or in, a hospital information system 120B. The hospital information system 120B may be a comprehensive, integrated information system designed to manage the administrative, financial and clinical aspects of a hospital or other medical installations such as small doctor's practices. The hospital information system 120B may be based on a network of server and client machines and help to organize the medical treatment comprising diagnostic tasks such as radiology or other examinations as well as treatment tasks. In some examples, the hospital information system 120B may comprise a number of subsystems distributed on a number of computer machines interconnected in a network which provide the required features and their components. The patient environment information obtaining device 120 may be embodied as a software residing in the hospital information system 120B or hardware dedicated to running said software. In an example, the patient environment information obtaining device 120 may be fully integrated into the hospital information system 120B. Similarly, the software may comprise an interactive element (e.g., a button) in the user interface, which may be activated by a user (e.g., a doctor) to access the patient environment information providing system 110 via the data diagnose support interface.

In a further example, the patient environment information obtaining device 120 may be embodied as, or in, a mobile device 120C. The mobile device 120C may include desktop computers, laptops, handhelds, mobile devices, mobile telephones, watches, or any other computerized devices. For example, the mobile device may be deployed in an ambulance, inside a hospital, in a local medical centre, etc.

It will be understood that the patient environment information obtaining device 120 may be embodied as, or in, other devices.

In some examples, the patient environment information providing system 110 may further comprise an authentication device (not shown) configured to be an interface through which the user (e.g., doctor) can authenticate to access the patient environment information providing system 110. The authentication device may determine whether the user is authorized to access the patient environment information providing system 110 to obtain the requested service. For example, the authorization service may compare a received user credential against known user credentials to determine a match. The user credential may be a username and an associated password, an authentication token, a license key, or another suitable authentication credential.

With the patient environment information providing system and the patient environment information obtaining device, the circumstances of an event may be better determined. For example, from the uploaded pictures, videos and/or text information, it is possible to determine e.g., how was the patient located in the car, how did the car look like after the crash, how was the position of patient after the ski accident, and/or in which directions have been arms, legs, etc. positioned. A movie of the accident showing details of the injury or a trusted testimony easily transferred to add up to the medical record could hold valuable information. So depending on the workflow the doctor would like to check if additional information is available by sending a request to the patient environment information providing system. In some examples, the additional information may become available after some time but still in time for the diagnose. The additional information would be very helpful or even relevant for correct diagnose if the decision would be more precise when knowing about the circumstances of the event.

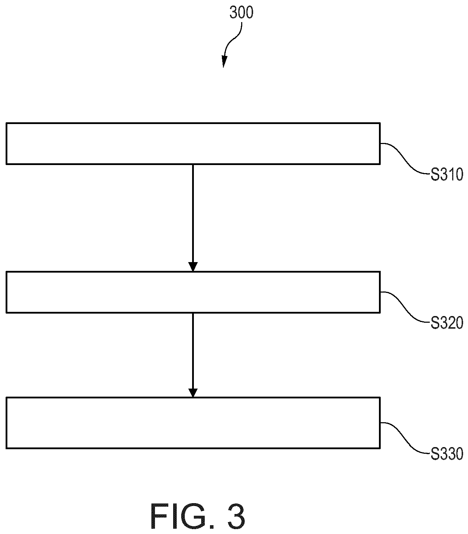

Reference is now made to a flowchart in Fig. 3 to describe a patient environment information providing method 300 as implemented by the patient environment information providing system. However, it will be understood that the steps described in the following are not necessarily tied to the architecture of the patient environment information providing system as described above but the following steps may also be understood as a teaching in its own right. The method is suitable for managing and collecting information about a circumstance of an event having an impact on a health status of a patient.

At step S310, a data provider access to the system is provided via at least one on-line user interface for inputting data that is relevant to the event from a plurality of information sources.

The at least one on-line user interface may comprise one or more of a browser-based web user interface, an app installable on a mobile device, and a web portal.

The input data may comprise location information, time information, and/or a short description. The input data may comprise first supportive data provided by a first aid provider. The input data may comprise pictures taken at a site of the event. The input data may comprise videos taken at a site of the event. The input data may comprise a voice recording. The input data may comprise weather information that is relevant to the event. The input data may comprise publicly available information about an event environment. In some further examples, the input data may comprise any combination of the above-described exemplary input data.

At step S320, the input data is analyzed to obtain synchronizing information and to synchronize the input data from the plurality of information sources to the event based on the synchronizing information to provide a support data set for the patient and the event.

The synchronization information may comprise location information and/or time information of the input data. The synchronization information may comprise image information on a picture and/or on a video. The synchronization information may comprise information on a license plate of a vehicle. The synchronization information may comprise a dedicated QR code on an ambulance, on a medical staff, on a police staff, and/or on a medical equipment. The synchronization information may comprise a wireless local network identifier. The synchronization may comprise a cell ID of a mobile phone network. The synchronization information may comprise a find-me token. In some further examples, the synchronization information may comprise any combination of the above-described exemplary synchronization information.

At step S330, a data diagnose support interface is provided to at least one patient environment information obtaining device to receive a data support request, and to provide the support data set for the patient and the event to the at least one patient environment information obtaining device, in response to receiving the data support request.

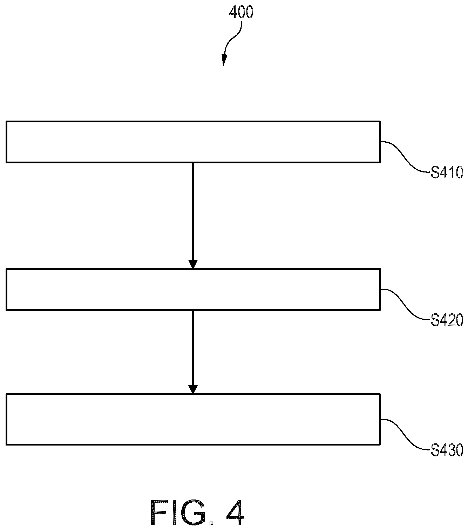

Reference is now made to flow chart in Fig. 4 to describe a patient environment information obtaining method 400 as implemented by the patient environment information obtaining device. However, it will be understood that the steps described in the following are not necessarily tied to the architecture of the patient environment information obtaining device as described above but the following steps may also be understood as a teaching in its own right.

At step S410, a user input is received via a user interface. The user input is indicative of a need for information about a circumstance of an event having an impact on a health status of a patient. For example, the user interface may comprise an interactive element. The interactive element may be activated by a user (e.g., a doctor) with a mouse, keyboard, finger, voice command, or other assistive technology. Once activated, the following function is performed.

At step S420, a data diagnose support request is sent to a patient environment information providing system via the data diagnose support interface. The data diagnose support request may comprise patient data of the patient and case data including information of the event.

At step S430, a support data set for the patient and the event from the system via the data diagnose support interface.

Some or all components of the patient environment information providing system and the patient environment information obtaining device may be arranged in hardware such as a suitably programmed FPGA or as hardwired IC chip.

One or more features of the patient environment information providing system and the patient environment information obtaining device disclosed herein may be configured or implemented as/with circuitry encoded within a computer-readable medium, and/or combinations thereof. Circuitry may include discrete and/or integrated circuitry, application ASIC, a SOC, and combinations thereof., a machine, a computer system, a processor and memory, a computer program.

In another exemplary embodiment of the present invention, a computer program or a computer program element is provided that is characterized by being adapted to execute the method steps of the method according to one of the preceding embodiments, on an appropriate system.

The computer program element might therefore be stored on a computer unit, which might also be part of an embodiment of the present invention. This computing unit may be adapted to perform or induce a performing of the steps of the method described above. Moreover, it may be adapted to operate the components of the above-described apparatus. The computing unit can be adapted to operate automatically and/or to execute the orders of a user. A computer program may be loaded into a working memory of a data processor. The data processor may thus be equipped to carry out the method of the invention.

This exemplary embodiment of the invention covers both, a computer program that right from the beginning uses the invention and a computer program that by means of an up-date turns an existing program into a program that uses the invention.

Further on, the computer program element might be able to provide all necessary steps to fulfill the procedure of an exemplary embodiment of the method as described above.

According to a further exemplary embodiment of the present invention, a computer readable medium, such as a CD-ROM, is presented wherein the computer readable medium has a computer program element stored on it which computer program element is described by the preceding section.

A computer program may be stored and/or distributed on a suitable medium (in particular, but not necessarily, a non-transitory medium), such as an optical storage medium or a solid-state medium supplied together with or as part of other hardware, but may also be distributed in other forms, such as via the internet or other wired or wireless telecommunication systems.

However, the computer program may also be presented over a network like the World Wide Web and can be downloaded into the working memory of a data processor from such a network. According to a further exemplary embodiment of the present invention, a medium for making a computer program element available for downloading is provided, which computer program element is arranged to perform a method according to one of the previously described embodiments of the invention.

It has to be noted that embodiments of the invention are described with reference to different subject matters. In particular, some embodiments are described with reference to method type claims whereas other embodiments are described with reference to the device type claims. However, a person skilled in the art will gather from the above and the following description that, unless otherwise notified, in addition to any combination of features belonging to one type of subject matter also any combination between features relating to different subject matters is considered to be disclosed with this application. However, all features can be combined providing synergetic effects that are more than the simple summation of the features.

While the invention has been illustrated and described in detail in the drawings and foregoing description, such illustration and description are to be considered illustrative or exemplary and not restrictive. The invention is not limited to the disclosed embodiments. Other variations to the disclosed embodiments can be understood and effected by those skilled in the art in practicing a claimed invention, from a study of the drawings, the disclosure, and the dependent claims.

In the claims, the word "comprising" does not exclude other elements or steps, and the indefinite article "a" or "an" does not exclude a plurality. A single processor or other unit may fulfill the functions of several items re-cited in the claims. The mere fact that certain measures are re-cited in mutually different dependent claims does not indicate that a combination of these measures cannot be used to advantage. Any reference signs in the claims should not be construed as limiting the scope

## Claims

1. A patient environment information providing system (110) for managing and collecting information about a circumstance of an event having an impact on a health status of a patient, the system comprising:
a data collection device (112);
a data synchronization device (114); and
a data diagnose support device (116);
wherein the data collection device is configured to provide a data provider access to the system via at least one on-line user interface for inputting data that is relevant to the event from a plurality of information sources;
wherein the data synchronization device is configured to analyze the input data to obtain synchronizing information and to synchronize the input data from the plurality of information sources to the event based on the synchronizing information to provide a support data set for the patient and the event; and
wherein the data diagnose support device is configured to provide a data diagnose support interface to at least one patient environment information obtaining device to receive a data diagnose support request therefrom, and in response to receiving the data diagnose support request, to provide the support data set for the patient and the event to the at least one patient environment information obtaining device.

2. The patient environment information providing system according to claim 1, further comprising:
a database configured to store a plurality of case data sets comprising information about a plurality of events having an impact on a health status of one or more patients, each data set comprising event information and patient information.

3. The patient environment information providing system according to claim 2,
wherein in response to receiving the data support request, the data diagnose support device is configured to search in the database for one or more case data sets that could match the received data support request, and to perform one or more plausibility checks on the one or more case data sets.

4. The patient environment information providing system according to any one of the preceding claims,
wherein the data synchronization device is further configured to analyze the input data to determine whether the input data contains personal information, and in response to determining that the input data contains personal information, the data synchronization device is configured to:
- not synchronize the input data containing personal information to the event;
- label the input data containing personal information which is only assessable when a privacy approval is available; or
- hide personal information contained in the input data, wherein the hidden personal information is only accessible when a privacy approval is available or for a certain authority.

5. The patient environment information providing system according to any one of the preceding claims,
wherein the data synchronization device is further configured to assign a level of reliability to the input data in accordance with information resources associated with the input data.

6. The patient environment information providing system according to any one of the preceding claims,
wherein the at least one on-line user interface comprises one or more of:
- a browser-based web user interface;
- an app installable on a mobile device; and
- a web portal.

7. The patient environment information providing system according to any one of the preceding claims,
wherein the information about the at least one on-line user interface is obtainable from:
- an image-based input providing information about the at least one on-line user interface in a visual, machine-readable form;
- a network-based input with auto popup screen on a mobile device; and
- a beacon-based input with auto popup screen on a mobile device.

8. The patient environment information providing system according to any one of the preceding claims,
wherein the input data comprises one or more of:
- text information of the event with location information, time information, and/or a short description;
- first supportive data provided by a first aid provider;
- a picture;
- a video;
- a voice recording;
- weather information that is relevant to the event; and
- publicly available information about an event environment.

9. The patient environment information providing system according to any one of the preceding claims,
wherein the synchronization information comprises one or more of:
- location information and/or time information of the input data;
- image information on a picture and/or on a video;
- information on a license plate of a vehicle;
- a dedicated Quick Response, QR, code on an ambulance, on a medical staff, on a police staff, and/or on a medical equipment;
- a wireless local network identifier;
- a cell ID of a mobile phone network; and
- an electronic finder.

10. The patient environment information providing system according to any one of the preceding claims,
wherein the data diagnose support request comprises patient data of the patient and case data including information of the event.

11. The patient environment information providing system according to any one of the preceding claims,
wherein the system is a cloud-based system.

12. A patient environment information obtaining device (120), comprising:
- a user interface (122);
- a data diagnose support interface (124); and
- a processor (126);
wherein the user interface is configured to receive a user input indicative of a need for information about a circumstance of an event having an impact on a health status of a patient; and
wherein the processor is configured to send, via the data diagnose support interface, a data diagnose support request to a system for managing and collecting information about a circumstance of an event having an impact on a health status of a patient, and to receive, via the data diagnose support interface, a support data set for the patient and the event from the system.

13. The patient environment information obtaining device according to claim 12, comprising one or more of:
- a medical imaging system;
- a hospital information system; and
- a mobile device.

14. A patient environment information providing method (300) for managing and collecting information about a circumstance of an event having an impact on a health status of a patient, the method comprising:
- providing (S310) a data provider access to the system via at least one on-line user interface for inputting data that is relevant to the event from a plurality of information sources;
- analyzing (S320) the input data to obtain synchronizing information and to synchronize the input data from the plurality of information sources to the event based on the synchronizing information to provide a support data set for the patient and the event; and
- providing (S330) a data diagnose support interface to at least one patient environment information obtaining device to receive a data support request, and to provide the support data set for the patient and the event to the at least one patient environment information obtaining device, in response to receiving the data support request.

15. A patient environment information obtaining method (400), the method comprising:
receiving (S410), via a user interface, a user input indicative of a need for information about a circumstance of an event having an impact on a health status of a patient;
sending (S420), via the data diagnose support interface, a data diagnose support request to a patient environment information providing system; and
receiving (S430), via the data diagnose support interface, a support data set for the patient and the event from the system.
